# EUROPEAN PATENT SPECIFICATION

(11) **EP 2 934 712 B1**
(45) Date of publication and mention of the grant of the patent: **18.03.2020**
(21) Application number: 13865276.3
(22) Date of filing: 29.11.2013
(51) Int. Cl.: B01D 3/14, C12P 7/08, C07C 29/80

(54) **PRODUCTION OF ETHANOL PRODUCTS**
HERSTELLUNG VON ETHANOLPRODUKTEN
PRODUCTION DE PRODUITS DE L'ÉTHANOL

(30) Priority: 21.12.2012 IN 3575MU2012
(43) Date of publication of application: 28.10.2015
(73) Proprietor: Praj Industries Limited, Pune 411021 Maharashtra (IN)
(72) Inventor: DESHPANDE, Ghansham Baburao, Pune 411021 Maharashtra (IN); RATHI, Shrikant Subhash, Pune 411021 Maharashtra (IN); DESHPANDE, Devdatta Krishna, Pune 411021 Maharashtra (IN); PIMPUTKAR, Vijaykumar Surendranath, Pune 411021 Maharashtra (IN); BHOSALE, Abhijit Anil, Pune 411021 Maharashtra (IN)
(74) Representative: Müller & Schubert
(86) International application number: PCT/IN2013/000724
(87) International publication number: WO 2014/097311

(56) References cited:
- CN-Y- 201 299 983
- RU-C1- 2 104 731
- US-A- 3 445 345
- US-A- 3 445 345
- US-A- 3 990 952
- US-A- 5 035 776
- US-A- 5 035 776
- US-A1- 2009 324 796

## Description

### FIELD OF THE INVENTION

The invention relates to a process and apparatus for the preparation of ethanol products and more particularly to low steam requiring distillation of ethanol from a feedstock containing ethanol of at least 3% by volume using a series of specialized columns and efficiently heat integrated processes. Further the invention relates to preparation of different grades of ethanol products having minimum levels of congeners suitable for potable use.

### BACKGROUND

Many alcoholic beverages are produced comprising one or more of the steps of [1] producing ethanol by fermentation of a carbohydrate-rich feedstock to produce a fermented wash having concentration of about 2 to 23% alcohol by volume [ABV]; [2] distilling the product of fermentation at elevated temperatures to produce ethanol products like rectified spirit, neutral spirit or absolute alcohol and; [3] aging the ethanol spirits until it possesses desired flavour, aroma, and colour characteristics. The commercial production of alcohol by distillation has been in widespread operation for many centuries.

The distillation is a known technique for purification of a liquid substance and involves vaporizing the substance at its boiling point, condensing the vapour and collecting the purified form as a condensate. Distillation is useful for separating a mixture when the components have different boiling points. Several kinds of distillation techniques for binary or multi-component mixtures are described and practiced in the art, for example: (1) simple, (2) vacuum or reduced pressure, (3) fractional and (4) steam distillation.

To produce rectified spirit from a fermented wash, the wash is distilled in the one or multiple distillation columns to produce a product with ethanol content of about 93% ABV. This spirit contains various congeners like fusel oils, higher alcohols, acids, etc which are carried over during the distillation process.

On the other hand, a double distillation process is carried out for manufacturing neutral spirit in which a fermented wash is distilled to about 95% ABV and is then subjected to a process of hydro-selection or hydro-extraction in which ethanol is again diluted with water between about three to about twelve times and redistilled to the azeotropic concentration of ethanol. This process helps in removing the congeners that are unwanted in neutral spirit.

The complete removal of water from ethanol gives anhydrous alcohol, which is widely used in industry as a solvent in the synthesis of paints, pharmaceutical intermediaries, cosmetics, perfumes, and other products. Anhydrous ethanol is also an important component in alternative fuels such as gasohol or it can also be used as an oxygenate in gasoline. To make anhydrous ethanol, the azeotrope of ethanol-water is broken to achieve the desired strength of more than 99% ABV by using one of the dehydration methods like: 1] molecular sieve dehydration, 2] azeotropic distillation, or 3] membrane dehydration. This makes traditional distillation to obtain anhydrous ethanol a costly process requiring high amounts of energy to obtain pure ethanol. Other processes that can be used to obtain anhydrous ethanol include extractive distillation and salt rectification. However, these processes require high energy input resulting in expensive anhydrous ethanol products. Currently, typically about 3-4 kilograms of steam is used to prepare about one litre of high quality ethanol product, leading to need of new processes and apparatuses more energy efficient to achieve less stream consumption and more economic to operate.

Several methods have been described in the art, for example, the patent US4784868 describes a method of producing a potable spirit which comprises obtaining ethanol substantially free of congeners and water and then fractionally distilling this mixture to produce substantially water-free potable ethanol. However, this process uses liquid CO₂ in dehydration process and is not practical and economic in many situations. Therefore, there is further need for effective and economic processes for the preparation of high quality ethanol products, especially absolute ethanol for a variety of applications from potable use to vehicular fuels.

US 5 035 776 A discloses a thermally-integrated extractive distillation process for recovering anhydrous ethanol from fermentation or synthetic feedstocks that has a distillation train of four columns. Two columns are preconcentrators operated in parallel. The remaining columns are an extractive distillation dehydrating tower, and an entrainer-recovery column. The two preconcentrators and the dehydrating tower are operated at three successively increasing pressures so that the condensing vapors of the overhead product of the dehydrating tower supply the necessary heat to the reboiler of the intermediate-pressure preconcentrator. The overhead vapors of this preconcentrator are, in turn, used to supply the required heat to the reboiler of the lowest-pressure preconcentrator. The bottom product from each preconcentrator is used to preheat the dilute feed. Additional energy savings are accomplished by the appropriate heat exchange between the various feeds, overheads, and bottoms.

### BRIEF DESCRIPTION

The present invention provides a process for separating ethanol from a fermented wash containing minimum of 2% ABV to various high quality ethanol products like anhydrous ethanol to extra neutral ethanol having minimum qualities of congeners with no remarkable odour or taste.

In one embodiment of the present invention, the process of separation of ethanol from fermented wash may be divided into seven stages: 1] primary distillation, 2] aldehyde distillation, 3] low boiler distillation, 4] rectification distillation, 5] dehydration, 6] high boiler distillation and 7] simmering distillation.

According to the present invention, a distillation method for separating ethanol from a dilute ethanol containing feedstock comprising: dividing the feedstock into two feed streams of unequal size; preheating both the larger and the smaller of said feed streams; introducing said preheated feed streams in parallel into a pair of analyzer columns; maintaining a higher pressure in first analyzer column receiving said larger feed stream than in second analyzer column receiving said smaller feed stream; withdrawing a first hydrous ethanol stream from the upper portion of said analyzer columns and feeding it to an aldehyde column to separate an aldehyde free ethanol stream at the bottom portion and a technical alcohol stream at the upper portion of the column; mixing about three to twelve parts of water to one part of said aldehyde free ethanol stream, preheating it and subjecting it to a low boiler column to separate a second hydrous ethanol stream from the lower portion and a technical alcohol stream from the upper portion of the column; preheating said second hydrous ethanol stream and subjecting it to a rectifier column to obtain a rectified ethanol stream; dehydrating said rectified ethanol stream using a dehydration unit to obtain an anhydrous ethanol stream; introducing said anhydrous ethanol stream to a high boiler column to separate a technical alcohol stream at the bottom portion and a final anhydrous ethanol stream at the upper portion of the column; and introducing said final anhydrous ethanol stream to a simmering column to obtain ethanol product streams by optionally adding water to said final anhydrous ethanol stream in various amounts.

According to the present invention, a distillation apparatus for preparation/ recovering ethanol from a dilute ethanol containing feedstock comprising, in combination: a provision for splitting the feedstock into two feed streams of unequal size; a provision for preheating both the larger and the smaller of said feed streams; a high pressure first analyzer column and a low pressure second analyzer column; a provision for introducing the larger of said feed streams into said first analyzer column and a provision for introducing the smaller of said feed streams into said second analyzer column; a first reboiler means for condensing alcohol vapours from dehydration unit to supply the heat required in said first analyzer column; a second reboiler means for condensing overhead vapours from a rectifier column downstream to supply the heat required in said first analyzer column; a third reboiler means for condensing overhead vapours from said first analyzer column to supply the heat required in said second analyzer column; a provision for withdrawing of first hydrous ethanol streams from an upper portion of said analyzer columns; a provision for preheating and introducing said hydrous ethanol streams into an aldehyde column; a provision for withdrawing of an aldehyde free ethanol stream from a bottom portion of said aldehyde column; a provision for preheating and introducing said aldehyde free ethanol stream into said rectifier column; a provision for withdrawing of a rectified ethanol stream from an upper portion of said rectifier column; a provision for introducing an elemental hydroxide to said rectified ethanol stream to adjust its pH; a provision for introducing said rectified ethanol stream into a dehydration unit; a provision for withdrawing of an anhydrous ethanol stream from said dehydration unit; a provision for introducing an inorganic oxidising agent to said anhydrous ethanol stream; means for introducing said anhydrous ethanol stream into a simmering column having a provision to optionally mix water; and a provision for withdrawing of ethanol product streams from said simmering column.

### BRIEF DESCRIPTION OF THE DRAWINGS

Particular examples of methods in accordance with this invention will now be described with reference to accompanying drawings, in which:

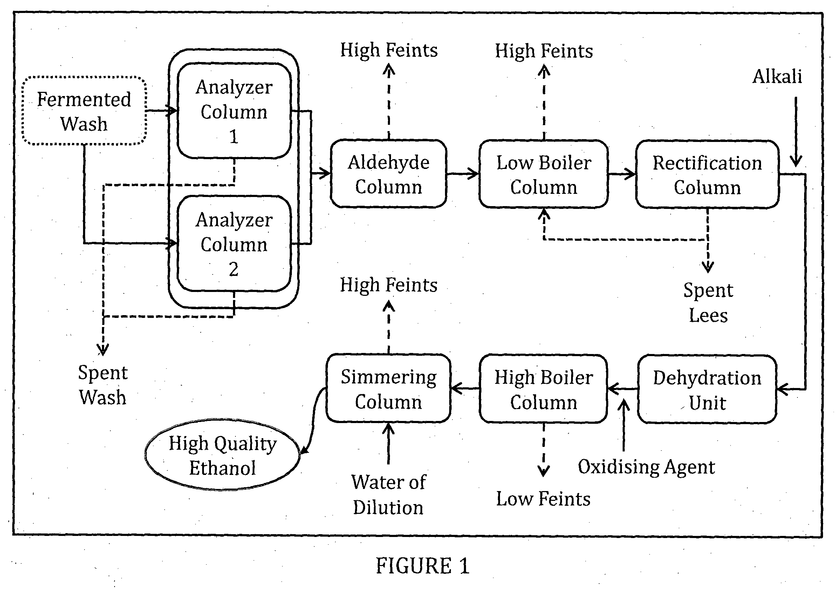

FIGURE 1 is a schematic diagram of the mass flow in the disclosed process. A fermented wash is split and fed to said analyzer columns. The distillate in then fed in a series to aldehyde column, low boiler column and rectification column. Then rectified stream is dehydrated in a dehydration unit and then subjected to high boiler column and at the end to a simmering column to finally get neutral spirit of desired quality. Different types of feints are obtained from different columns forming technical alcohol products of different grades.

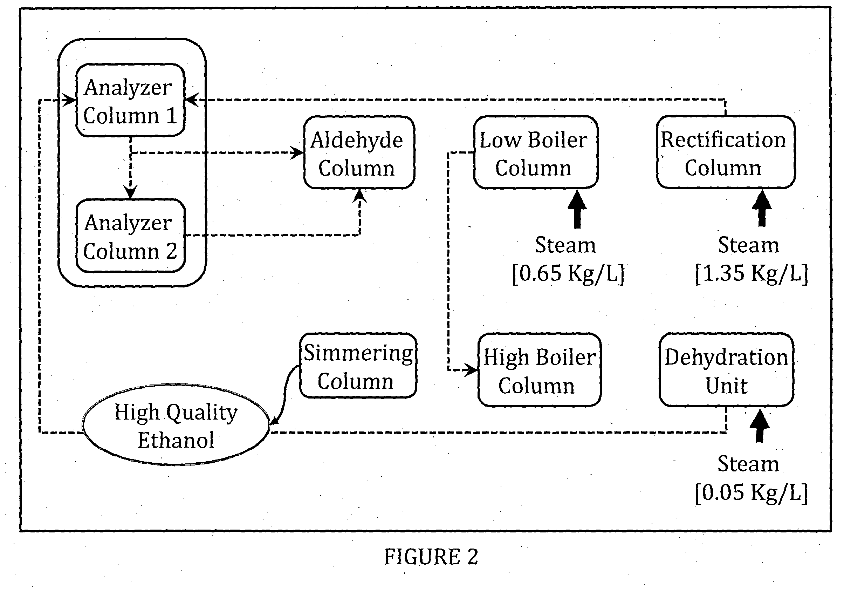

FIGURE 2 is a block diagram of the energy flow in the disclosed invention. The first analyzer column gets energy in the form of unused [excess] energy from rectification column and dehydration unit. The second analyzer column gets energy in the form of unused energy from said first analyzer column. The aldehyde analyzer column gets energy in the form of unused energy from both the said analyzer columns. The unused energy of said low boiler column is supplied to said high boiler column. In addition, fresh energy in the form of steam is supplied to said low boiler column, said rectification column and said dehydration unit at need.

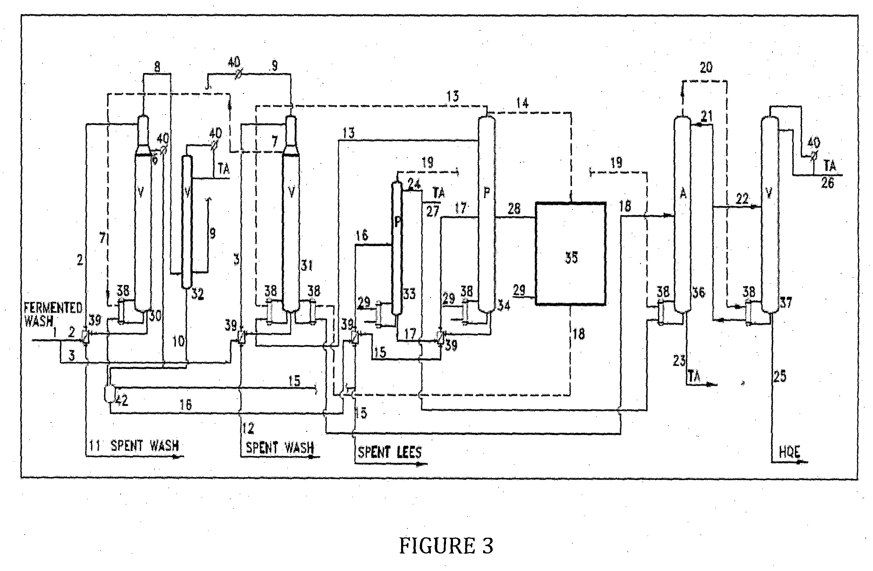

FIGURE 3 is an exemplary plan of the invention showing several features that control the process of distillation of ethanol from a fermented wash. It also depicts the efficient energy integration achieved through reuse of the heat energy left unused in the downstream steps to run the unit operations at upstream steps. The whole system may be divided into seven units, namely: 1] primary distillation unit, 2] aldehyde distillation unit, 3] low boiler distillation unit, 4] rectification distillation unit, 5] dehydration unit, 6] high boiler distillation unit, and 7] simmering unit. The unused energy left in the form of vapours from said rectification distillation unit is integrated to run said first analyzer column in said primary distillation unit. Further the unused energy left in the form of vapours from said dehydration unit is integrated with said first analyzer column. The unused energy left in the form of vapours from said low boiler distillation unit is integrated with said high boiler distillation unit. The unused energy left in the form of vapours from said primary distillation unit is integrated with said aldehyde column.

### DETAILED DESCRIPTION

In one embodiment of the present invention, a fermented wash with ethanol concentration of between 2 to 23% ABV, preferably 6 to 14% ABV is obtained from yeast fermentation of a carbohydrate rich feedstock like grains, cereals, tubers, molasses, sugarcane juice, lignocellulosic materials or synthetic ethanol. This wash is then split into two feed streams of unequal size, a larger feed stream comprising about 55 to 70% of the total wash and a smaller feed stream comprising remaining about 30 to 45% of the wash. These two feed streams have a temperature of about 30 °C. Before subjecting it to the primary distillation unit these feed streams are preheated to about 76 °C by plate-type heat exchangers. The said heat exchangers receive high energy spent wash of corresponding analyzer columns as heat donating liquid to preheat said fermented wash before subjecting it to the process of primary distillation. The said primary distillation unit comprises two analyzer columns operating at different working pressures. The first analyzer column receiving said larger feed stream operates at a higher pressure and temperature than the second analyzer column receiving said smaller feed stream. In this type of split primary distillation process said first analyzer column typically provide unused energy in the form of vapours to said second analyzer column, which is operated at relatively lower pressure and temperature than said first analyzer column. This split distillation using minimum amount of energy to separate ethanol from said fermentation wash forms an element of novelty in view of the total energy consumption per unit of final ethanol product produced. The ethanol containing streams obtained from the upper portions of said analyzer columns are combined to form first hydrous ethanol streams. The spent wash obtained from said analyzer columns possess excess unused energy, which in used in said heat exchangers to preheat said feed stream as described herein. The said analyzer columns of the invention are also equipped with degassing columns at the top, which are heat integrated by the vapours of said analyzer columns. It is understood in the art the utility of degassing column and hence not specifically depicted herein. These degassing columns remove dissolved gases like CO₂ present in fermented wash before its entry into said analyzer columns.

In another embodiment of the present invention, the process of preparation of the high quality ethanol as disclosed herein comprises of seven steps, namely: 1] primary distillation unit, 2] aldehyde distillation unit, 3] low boiler distillation unit, 4] rectification distillation unit, 5] dehydration unit, 6] high boiler distillation unit, and 7] simmering unit. The said primary distillation unit is described herein above is also called split distillation and it forms the first important step of the disclosed invention. In second step, a part of said first hydrous ethanol stream is subjected to an aldehyde distillation leading to the formation of an aldehyde free ethanol stream. The said aldehyde distillation process removes one or more aldehyde components of the stream making it free of any aldehyde taste or odour. Herein high feints obtained form a grade of technical alcohol used for a variety of non-food purposes. In third step, said aldehyde free ethanol stream along with the vapour condensate from both the analyzer columns is subjected to a low boiler distillation leading to the formation of a second hydrous ethanol stream. Before subjecting said aldehyde free ethanol stream to said low boiler distillation, it is diluted with about three to twelve parts of water or spent lees obtained downstream. Herein said low boiler distillation process removes one or more low boiling components than ethanol of the stream making it free of any low boilers like ethyl acetate and acetaldehyde and its associated taste or odour. Herein high feints obtained forms another grade of technical alcohol used for a variety of non-food purposes. In fourth step, said second hydrous ethanol stream is subjected to a rectification distillation leading to the formation of a rectified ethanol stream. Herein said rectified ethanol stream is treated with an elemental hydroxide like sodium hydroxide, potassium hydroxide, calcium hydroxide or equivalent other hydroxide to improve the pH of said stream to between pH 7 to pH 11. This treatment substantially improves the quality of said stream eradicating any acidity associated with it. The said rectification distillation process improves the ethanol content of the stream to about 93% ABV. This step further removes the congeners like acids and fusel oils present in the stream leading to a high quality of ethanol in said rectified ethanol stream. Herein high feints obtained forms another grade of technical alcohol, while the spent lees obtained are used as diluents in the third step described herein above. In fifth step, said rectified ethanol stream is subjected to a dehydration process leading to the formation of an anhydrous ethanol stream. This dehydration process improves the ethanol content of the stream to about 99% ABV. This dehydration is achieved by means of one or more of methods like membrane separation, molecular sieve dehydration, extractive distillation or adsorption separation of water or combination thereof. In sixth step, said anhydrous ethanol stream is subjected to a high boiler distillation leading to the formation of a final anhydrous ethanol stream. Herein said final anhydrous ethanol stream is treated with an inorganic oxidising agent like potassium permanganate, hydrogen peroxide, sodium percarbonate or equivalent chemical to remove other alcoholic impurities of higher primary and secondary alcohols like butanol, propanol, pentanol, ketones and other chemicals that adversely affect the organoleptic properties of the final products, leading to substantially improvement in the final quality of said stream. Herein further said high boiler distillation process removes one or more high boiling components than ethanol of the stream making it free of any high boilers like butanol and its associated taste or odour. Herein low feints obtained forms another grade of technical alcohol. In seventh step, said final anhydrous ethanol stream is subjected to a simmering distillation leading to the formation of a final ethanol product. Herein said simmering distillation process removes methanol and one or more sulphide components from the stream making it free of any associated taste or odour. The said simmering column has copper internal components, while other process equipments are made of stainless steel or other suitable materials. Herein high feints obtained forms another grade of technical alcohol. In the simmering process of the disclosed invention, various amount of water of dilution may be added before or after the production process to achieve different types of ethanol products based on the final content of ethanol. Another novelty of the process is that the final iso-propanol amount is reduced at least to 40% of the initial amount in the feed streams.

In yet another embodiment of the invention, in said primary split distillation unit, said second analyzer column operating at lower pressure operates at a pressure between about 0.1 to about 1 bar[a], preferably about 0.2 bar[a]. Any unused energy left in said first analyzer column is used to drive said second analyzer column and said aldehyde column. In said rectification distillation unit, said rectification column operates at a pressure between about 1 to about 10 bar[a], preferably between about 2 to about 3 bar[a]. Herein the column pressure is so adjusted that it acts as the heat source for the first analyzer column operating upstream in the process.

In further embodiment of the disclosed invention as illustrated in FIGURE 3 the steps of said process are: 1] the fermented wash (1) is divided unequally into two feed streams (2 and 3), each feed stream is then preheated to desired temperature by heat exchangers (39) and enters into said first analyzer column (31) and said second analyzer column (30); 2] then a part of first hydrous ethanol stream (8) from said second analyzer column (30) is directed to said aldehyde column (32), while remaining part (6) is condensed (40) and directed (6) to the dilution tank (42), similarly first hydrous ethanol stream (9) from said first analyzer column (30) is directed to the column (32); 3] said aldehyde free ethanol stream (10) is directed the dilution tank (42), in the dilution tank the stream is dilution to three to twelve times with water or spent lees (15) and this stream (16) is directed to said low boiler column (33); 4] said second hydrous ethanol stream (17) obtained from said low boiler column (33) is preheated in a heat exchanger (39) by the energy of spent lees (15) of the column (34) and is directed to said rectification column (34) to obtain said rectified ethanol stream (28); 5] said rectified ethanol stream (28) is then directed to said dehydration unit (35) to obtain said anhydrous ethanol stream (18); 6] said anhydrous ethanol stream (18) is then directed to said high boiler column (36) to obtain said final anhydrous ethanol stream (22); and 7] said final anhydrous ethanol stream (22) is then directed to said simmering column (37) to obtain said high quality ethanol products (25). Here said first analyzer column (31) is heat integrated with the unused energy in the form of vapours of a part of alcohol vapours (13) from said rectification column (34). Further said second analyzer column (30) is heat integrated with the unused energy in the form of vapours of alcohol (7) from said first analyzer column (31). Further said first analyzer column (31) is heat integrated with the unused energy in the form of vapours of dehydrated alcohol (18) from said dehydration unit (35). Further said high boiler column (36) is heat integrated with the unused energy in the form of vapours of alcohol (19) from said low boiler column (33). Further again said simmering column (37) is heat integrated with the unused energy in the form of vapours of alcohol (20) from said high boiler column (36). The fresh energy in the form of steam is given to units (33), (34) and (35) from an external heat source (29). The heat integration of unused energy is said process is done using reboilers (38). Reboilers are a type of heat exchangers that can exchange heat from a high energy liquid or vapours to low energy liquids. In the disclosed process the columns 30, 31, 32 and 37 are under desired vacuum, while columns 33 and 34 are above atmospheric pressure; and column 36 is at atmospheric pressure. Technical grade alcohol products [TA] are obtained from columns 32, 33, 36 and 37.

Examples provided below give wider utility of the invention without any limitations as to the variations that may be appreciated by a person skilled in the art. A non-limiting summary of various embodiments is given in the examples and tables, which demonstrate the advantageous and novel aspects of the process disclosed herein. Particular examples of processes in accordance with this invention will now be described with reference to the accompanying drawings

### EXAMPLE 1

In one embodiment of the present invention about 1000 kilolitres of fermented wash produced by yeast from a carbohydrate containing feedstock was taken, which contained about 10% v/v of ethanol. This fermented wash was split to two unequal feed streams of about 55% and 45% of initial amounts of the wash. The larger feed stream was preheated to about 76 °C by a plate type of heat exchanger receiving unused energy in the form of liquid obtained from processes downstream of analyzer columns, and fed into a first analyzer column operating at a higher pressure receiving unused energy of the rectification column downstream. The smaller feed stream was preheated to about 49 °C by a plate type of heat exchanger receiving unused energy in the form of liquid from processes downstream, and fed into a second analyzer column operating at a lower pressure. Before feeding these streams to the analyzer columns, the stream were passed through degassing column located above the analyzer column in order to degas said feed streams before entry to said analyzer columns. The distilled out ethanol streams from each column were combined and formed a first hydrous ethanol stream. This split primary distillation produced typically about 55% v/v ethanol content in said first hydrous ethanol stream. The second analyzer column was heat integrated by energy in the form of vapours left unused in said first analyzer column operating at higher pressure than said second analyzer column, which operated at a low vacuum condition. In the next step, said a part of first hydrous ethanol stream said second analyzer column was subjected to an aldehyde column, which received unused energy in the form of vapours from said first analyser column. In this aldehyde distillation, aldehyde congeners of said first hydrous ethanol stream were removed leading to the formation of an aldehyde free ethanol stream. In the next step, said aldehyde free ethanol stream along with the vapour condensate from both the analyzer columns was diluted to about 1:3 ethanol to water dilution ratio on volume basis and subjected to a low boiler column, which received fresh energy in the form of steam at need and dilution water left from the rectification column present downstream was used as dilution water. Further said aldehyde free ethanol stream was preheated to about 92 °C by a heat exchanger receiving unused energy left in the rectification lees. In this low boiler distillation, low boiling congeners of said aldehyde free ethanol stream were removed leading to the formation of a second hydrous ethanol stream. In further step, said second hydrous ethanol stream was subjected to a rectification column, which received fresh energy in the form of steam at need. Here, said second hydrous ethanol stream was preheated to about 108 °C by a heat exchanger receiving unused energy left in the rectification lees. In this rectification distillation, the alcohol content of said second hydrous ethanol stream was increased up to 93% v/v of ethanol to get a rectified ethanol stream. In the next step, said rectified ethanol stream [which is in vapour form] was subjected to a dehydration unit, which received fresh energy in the form of steam at need. By dehydration the ethanol content of said rectified ethanol stream was increased to more than 99% v/v of ethanol to obtain an anhydrous ethanol stream. In further step, said anhydrous ethanol stream was subjected to a high boiler column, which received unused energy in the form of vapours from said low boiler column. In this high boiler distillation, high boiling congeners of said anhydrous ethanol stream were removed leading to the formation of a final anhydrous ethanol stream. Then said final anhydrous ethanol stream was subjected to a simmering column to obtain finer grades of ethanol product having improved congener profiles. The said final anhydrous ethanol stream formed an ethanol product called high quality ethanol [HQE] of no odour or taste of any of congeners and suitable for various application like making potable grade ethanol, perfumery and pharmaceutical grade ethanol with other alcohol products. Also different streams of impure ethanol streams were produced having high ethanol as well as high congener content from aldehyde column, low boiler column, high boiler column and simmering column, which were termed technical ethanol streams and subjected to the preparation of technical alcohol products. Finally about 95 kilolitres of HQNS was obtained from about 1000 kilolitres of fermented was with 10% v/v of ethanol with consumption of about 2 kilograms of steam per litre of HQE.

### EXAMPLE 2

In another embodiment of the present invention about 1000 kilolitres of fermented wash produced by yeast from a carbohydrate containing feedstock was taken, which contained about 6% v/v of ethanol. This fermented wash was split to two unequal feed streams of about 55% and 45% of initial amounts of the wash. The larger feed stream was preheated to about 76 °C by a plate type of heat exchanger receiving unused energy in the form of liquid obtained from processes downstream of analyzer columns, and fed into a first analyzer column operating at a higher pressure receiving unused energy of the rectification column downstream. The smaller feed stream was preheated to about 49 °C by a plate type of heat exchanger receiving unused energy in the form of liquid from processes downstream, and fed into a second analyzer column operating at a lower pressure. Before feeding these streams to the analyzer columns, the stream were passed through degassing column located above the analyzer column in order to degas said feed streams before entry to said analyzer columns. The distilled out ethanol streams from each column were combined and formed a first hydrous ethanol stream. This split primary distillation produced typically about 43% v/v ethanol content in said first hydrous ethanol stream. The second analyzer column was heat integrated by energy in the form of vapours left unused in said first analyzer column operating at higher pressure than said second analyzer column, which operated at a low vacuum condition. In the next step, said a part of first hydrous ethanol stream was subjected to an aldehyde column, which received unused energy in the form of vapours from said first analyser column. In this aldehyde distillation, aldehyde congeners of said first hydrous ethanol stream were removed leading to the formation of an aldehyde free ethanol stream. In the next step, said aldehyde free ethanol stream along with the vapour condensate from both the analyzer columns was diluted to about 1:3 ethanol to water dilution ratio on volume basis and subjected to a low boiler column, which received fresh energy in the form of steam at need and dilution water left from the rectification column present downstream was used as dilution water. Further said aldehyde free ethanol stream was preheated to about 92 °C by a heat exchanger receiving unused energy left in the rectification lees. In this low boiler distillation, low boiling congeners of said aldehyde free ethanol stream were removed leading to the formation of a second hydrous ethanol stream. In further step, said second hydrous ethanol stream was subjected to a rectification column, which received fresh energy in the form of steam at need. Here, said second hydrous ethanol stream was preheated to about 108 °C by a heat exchanger receiving unused energy left in the rectification lees. In this rectification distillation, the alcohol content of said second hydrous ethanol stream was increased up to 93% v/v of ethanol to get a rectified ethanol stream. In the next step, said rectified ethanol stream [which is in vapour form] was subjected to a dehydration unit, which received fresh energy in the form of steam at need. Herein before said dehydration step said rectified ethanol stream was treated with an elemental hydroxide like sodium hydroxide, potassium hydroxide, calcium hydroxide or equivalent other hydroxide to improve the pH of said stream to between pH 7 to pH 11. Thereby the formation of compounds like acetal due to reaction of acetaldehyde and ethanol is diminished.This treatment substantially improved the quality of said stream eradicating any acidity associated with it. By dehydration the ethanol content of said rectified ethanol stream was increased to more than 99% v/v of ethanol to obtain an anhydrous ethanol stream. Further said anhydrous ethanol stream was treated with an inorganic oxidising agent like potassium permanganate, hydrogen peroxide, sodium percarbonate or equivalent chemical [added up to 30 PPM concentration] to remove other organic impurities of higher primary and secondary alcohols like butanol, propanol, pentanol, ketones, aldehydes and other chemicals that adversely affect the organoleptic properties of the final products, leading to substantially improvement in the final quality of said stream. This treatment further improved the UV absorbance spectrum of said stream between 220 nm to 270 nm, qualifying said stream for more stringent specifications on said impurities in the final products. In further step, said anhydrous ethanol stream was subjected to a high boiler column, which received unused energy in the form of vapours from said low boiler column. In this high boiler distillation, high boiling congeners of said anhydrous ethanol stream were removed leading to the formation of a final anhydrous ethanol stream. Then said final anhydrous ethanol stream was subjected to a simmering column to obtain finer grades of ethanol product having improved congener profiles. The said final anhydrous ethanol stream formed an ethanol product called high quality neutral spirit [HQE] of no odour or taste of any of congeners and suitable for various application like making potable grade ethanol with other alcohol products. Also different streams of impure ethanol streams were produced having high ethanol as well as high congener content from aldehyde column, low boiler column, high boiler column and simmering column, which were termed technical ethanol streams and subjected to the preparation of technical alcohol products. Finally about 57 kilolitres of HQNS was obtained from about 1000 kilolitres of fermented was with 6% v/v of ethanol with consumption of about 2.2 kilograms of steam per litre of HQE.

### EXAMPLE 3

In one embodiment of the present invention about 1000 kilolitres of fermented wash produced by yeast from a carbohydrate containing feedstock was taken, which contained about 14% v/v of ethanol. This fermented wash called feed stream was preheated to about 76 °C by a plate type of heat exchanger receiving unused energy in the form of liquid obtained from processes downstream and fed into a first analyzer column operated by unused energy of the rectification column downstream. Before feeding this streams to the analyzer column, it were passed through degassing column located above the analyzer column in order to degas said feed stream before entry to said analyzer column. The distilled out ethanol stream formed a first hydrous ethanol stream. This primary distillation produced typically about 62% v/v ethanol content in said first hydrous ethanol stream. The second analyzer column was heat integrated by energy in the form of vapours left unused in said first analyzer column operating. In the next step, said a part of first hydrous ethanol stream was subjected to an aldehyde column, which received unused energy in the form of vapours from said first analyser column. In this aldehyde distillation, aldehyde congeners of said first hydrous ethanol stream were removed leading to the formation of an aldehyde free ethanol stream. In the next step, said aldehyde free ethanol stream along with the vapour condensate from both the analyzer columns was diluted to about 1:3 ethanol to water dilution ratio on volume basis and subjected to a low boiler column, which received fresh energy in the form of steam at need and dilution water left from the rectification column present downstream was used as dilution water. Further said aldehyde free ethanol stream was preheated to about 92 °C by a heat exchanger receiving unused energy left in the rectification lees. In this low boiler distillation, low boiling congeners of said aldehyde free ethanol stream were removed leading to the formation of a second hydrous ethanol stream. In further step, said second hydrous ethanol stream was subjected to a rectification column, which received fresh energy in the form of steam at need. Here, said second hydrous ethanol stream was preheated to about 108 °C by a heat exchanger receiving unused energy left in the rectification lees. In this rectification distillation, the alcohol content of said second hydrous ethanol stream was increased up to 93% v/v of ethanol to get a rectified ethanol stream. Herein said rectified ethanol stream was treated with an elemental hydroxide like sodium hydroxide, potassium hydroxide, calcium hydroxide or equivalent other hydroxide to improve the pH of said stream to between pH 7 to pH 11. This treatment substantially improved the quality of said stream eradicating any acidity associated with it. In the next step, said rectified ethanol stream [which is in vapour form] was subjected to a dehydration unit, which received fresh energy in the form of steam at need. By dehydration the ethanol content of said rectified ethanol stream was increased to more than 99% v/v of ethanol to obtain an anhydrous ethanol stream. In further step, said anhydrous ethanol stream was subjected to a high boiler column, which received unused energy in the form of vapours from said low boiler column. Further said anhydrous ethanol stream was treated with an inorganic oxidising agent like potassium permanganate, hydrogen peroxide, sodium percarbonate or equivalent chemical [added up to 30 PPM concentration] to remove other organic impurities of higher primary and secondary alcohols like butanol, propanol, pentanol, ketones, aldehydes and other chemicals that adversely affect the organoleptic properties of the final products, leading to substantially improvement in the final quality of said stream. This treatment further improved the UV absorbance spectrum said stream of between 220 nm to 270 nm, qualifying said stream for more stringent specifications on said impurities in the final products. In this high boiler distillation, high boiling congeners of said anhydrous ethanol stream were removed leading to the formation of a final anhydrous ethanol stream. Then said final anhydrous ethanol stream was subjected to a simmering column to obtain finer grades of ethanol product having improved congener profiles. The said final anhydrous ethanol stream formed an ethanol product called high quality neutral spirit [HQE] of no odour or taste of any of congeners and suitable for various application like making potable grade ethanol with other alcohol products. Also different streams of impure ethanol streams were produced having high ethanol as well as high congener content from aldehyde column, low boiler column, high boiler column, and simmering column, which were termed technical ethanol streams and subjected to the preparation of technical alcohol products. Finally about 133 kilolitres of HQNS was obtained from about 1000 kilolitres of fermented was with 14% v/v of ethanol with consumption of less than 2 kilograms of steam per litre of HQE.

### EXAMPLE 4

By the process of present invention, different types and grades of ethanol products were obtained. An illustrative typical chemical composition of HQE is listed in TABLE 1. The amount of different congeners is given in parts per million by volume.

**TABLE 1: Chemical composition of HQE**

| **Sr No** | **Component** | **Composition** |
|---|---|---|
| 1 | Ethanol | 93-99.9% V/V |
| 2 | Acetaldehyde | Less than 0.1 PPM |
| 3 | Methyl acetate | Less than 0.1 PPM |
| 4 | Ethyl acetate | Less than 0.1 PPM |
| 5 | Methanol | Less than 0.1 PPM |
| 6 | 2-Butanol | Less than 0.1 PPM |
| 7 | N-Propanol | Less than 0.1 PPM |
| 8 | Iso-butanol | Less than 0.1 PPM |
| 9 | Iso-amyl alcohol (Iso-pentanol) | Less than 0.1 PPM |
| 10 | N-Amyl Alcohol | Less than 0.1 PPM |
| 11 | Acetic acid | Less than 0.1 PPM |
| 12 | Furfural | Less than 0.1 PPM |
| 13 | 2,3 Pentanedione | Less than 0.001 PPM |
| 14 | 2,3 Butanedione | Less than 0.001 PPM |
| 15 | Acetal | Less than 0.1 PPM |
| 16 | Isopropanol | Less than 0.1 PPM |
| 17 | Dimethyl disulphide | Less than 0.1 PPM |
| 18 | Water | Balance |

While the invention has been particularly shown and described with reference to embodiments listed in examples, it will be appreciated that several of the above disclosed and other features and functions, or alternatives thereof, may be desirably combined into many other different systems or applications. Also that various presently unforeseen and unanticipated alternatives, modifications, variations, or improvements therein may be subsequently made by those skilled in the art which are also intended to be encompassed by the following claims. Although the invention has been described with reference to specific preferred embodiments, it is not intended to be limited thereto, rather those having ordinary skill in the art will recognize that variations and modifications may be made therein which are within the spirit of the invention and within the scope of the claims.

## Claims

1. A distillation method for separating ethanol from a dilute ethanol containing feedstock comprising:
(a) dividing the feedstock into two feed streams of unequal size;
(b) preheating both the larger and the smaller of said feed streams;
(c) introducing said preheated feed streams in parallel into a pair of analyzer columns;
(d) maintaining a higher pressure in first analyzer column receiving said larger feed stream than in second analyzer column receiving said smaller feed stream;
(e) withdrawing first hydrous ethanol streams from the upper portion of said analyzer columns and feeding it to an aldehyde column to separate an aldehyde free ethanol stream at the bottom portion and a technical alcohol stream at the upper portion of the column;
(f) mixing between about three to about twelve parts of water or spent lees to one part of ethanol in said aldehyde free ethanol stream, preheating it and subjecting it to a low boiler column to separate a second hydrous ethanol stream from the lower portion and a technical alcohol stream from the upper portion of the column;
(g) preheating said second hydrous ethanol stream and subjecting it to a rectifier column to obtained a rectified ethanol stream, wherein said rectified ethanol stream is treated with an elemental hydroxide to adjust its pH to between pH 7 and pH 11;
(h) dehydrating said rectified ethanol stream using a dehydration unit to obtain an anhydrous ethanol stream, wherein said dehydration of rectified ethanol stream is achieved by means of a membrane separation technique, molecular sieve dehydration, or adsorption separation of water or a combination thereof;
(i) introducing said anhydrous ethanol stream to a high boiler column to separate a technical alcohol stream at the bottom portion and a final anhydrous ethanol stream at the upper portion of the column; wherein said anhydrous ethanol stream is treated with an inorganic oxidising agent up to 30 PPM concentration, and
(j) introducing said final anhydrous ethanol stream to a simmering column to obtain ethanol product streams by optionally adding water to said final anhydrous ethanol stream in various amounts.

2. The method of claim 1, wherein said larger feed stream comprises between 50 to 70% by weight of the total feedstock.

3. The method of claim 1, wherein said aldehyde column separates at least one aldehyde component present in said first hydrous ethanol streams obtained from said analyzer columns.

4. The method of claim 1, wherein said low boiler column separates at least one low boiling component present in said aldehyde free ethanol stream obtained from said aldehyde column.

5. The method of claim 1, wherein said rectifier column further purifies said second hydrous ethanol stream obtained from said low boiler column of at least one high boiling or one low boiling component or both.

6. The method of claim 1, wherein said high boiler column separates at least one high boiling component present in said anhydrous ethanol stream obtained from said dehydration unit.

7. The method of claim 1, wherein water is optionally added in the final step in various amounts to obtained ethanol products having about between 0.1 % to 6 % water by volume.

8. The method of claim 1, wherein not more than about 2 kilograms of steam is required to obtain about one litre of an ethanol product.

9. The method of claim 1, wherein heat left unused in said rectifier column is integrated with said first analyzer column.

10. The method of claim 1, wherein heat left unused in said dehydration unit is integrated with said first analyzer column.

11. The method of claim 1, wherein heat left unused in said first analyzer column is integrated with said aldehyde or said second analyzer column.

12. The method of claim 1, wherein heat left unused in said low boiler column is integrated with said high boiler column.

13. The method of claim 1, wherein fresh energy in the form of steam is supplied to said low boiler column, said rectifier column and said dehydration unit at need.

14. The method of claim 1, wherein preheating at various steps is achieved by integrating heat left unused in other unit steps.

15. The method of claim 1, wherein said dilute ethanol containing feedstock comprises: a fermented wash or synthetic ethanol feed or a combination thereof.

16. The method of claim 1, wherein amount of ethanol in said dilute ethanol containing feedstock is at least 3 percent by volume.

17. The method of claim 1, wherein said technical alcohol streams are obtained from said aldehyde column, said low boiler column, said high boiler column and said simmering column.

18. The method of claim 1, wherein said rectified ethanol stream obtained from said rectifier column contains at least 93% of ethanol by volume.

19. The method of claim 1, wherein said anhydrous ethanol stream obtained from said dehydration unit contains at least 99% of ethanol by volume.

20. The method of claim 1, wherein said ethanol product streams obtained from said simmering column comprises methanol not more than 1 PPM and aldehyde not more than 1 PPM.

21. The method of claim 1, wherein said ethanol product streams obtained from said simmering column comprises ethyl acetate not more than 1 PPM and fusel oils not more than 2 PPM.

22. The method of claim 1, wherein ethanol product streams obtained from said simmering column is neutral in odour or taste.

23. The method of claim 1, wherein in said first hydrous ethanol streams obtained from said analyzer columns the amount of isopropyl alcohol is reduced to at least 40% of the initial amount present in said feed streams.

24. A distillation apparatus for recovering ethanol from a dilute ethanol containing feedstock, for use in a method of any of claims 1 to 23, comprising:
(a) a feedstock splitting unit to get two feed streams of unequal size;
(b) a preheating unit to heat both the larger and the smaller of said feed streams;
(c) a first analyzer column and a second analyzer column, an aldehyde column, a low boiler column, a rectifier column, a dehydration unit, a high boiler column, and a simmering column;
(d) a provision for introducing the larger of said feed streams into said first analyzer column and provision for introducing the smaller of said feed streams into said second analyzer column;
(e) a first reboiler for condensing alcohol vapours from the dehydration unit downstream to supply the heat required in said first analyzer column;
(f) a second reboiler for condensing overhead vapours from a rectifier column downstream of said analyzer columns to supply the heat required in said first analyzer column;
(g) a third reboiler for condensing overhead vapours from said first analyzer column to supply the heat required in said second analyzer column;
(h) a provision for withdrawing of first hydrous ethanol streams from an upper portion of said analyzer columns;
(i) a provision for preheating and introducing said first hydrous ethanol streams into an said aldehyde column;
(j) a provision for withdrawing of an aldehyde free ethanol stream from a bottom portion of said aldehyde column;
(k) a dilution tank for dilution of said aldehyde free ethanol stream with water or spent lees;
(l) a provision for preheating and introducing said diluted aldehyde free ethanol stream into said rectifier column;
(m) a provision for withdrawing a second hydrous ethanol stream from the lower portion and for withdrawing a technical alcohol stream from the upper portion of said low boiler column;
(n) a provision for preheating and introducing said second hydrous ethanol stream into said rectifier column;
(o) a provision for withdrawing of a rectified ethanol stream from an upper portion of said rectifier column;
(p) a provision for introducing an elemental hydroxide to said rectified ethanol stream to adjust its pH to between 7 and 11;
(q) a provision for introducing said rectified ethanol stream into a said dehydration unit, wherein said dehydration unit is one or more of a membrane separation unit, a molecular sieve unit or an adsorption unit;
(r) a provision for withdrawing of an anhydrous ethanol stream from said dehydration unit;
(s) a provision for introducing said anhydrous ethanol stream into said high boiler column;
(t) a provision for introducing an inorganic oxidising agent to said anhydrous ethanol stream;
(u) a provision for separating a technical alcohol stream at the bottom portion and a final anhydrous ethanol stream at the upper portion of said high boiler column;
(v) a provision for introducing said anhydrous ethanol stream into a simmering column having means to optionally mix water; and
(w) a provision for withdrawing of ethanol product streams from said simmering column.

25. The apparatus of claim 24, wherein heat left unused in said low boiler column is supplied to said high boiler column.

26. The apparatus of claim 24, wherein heat left unused in said high boiler column is supplied to said rectifier column.

27. The apparatus of claim 24, wherein heat left unused in said rectifier column is supplied to said first analyzer column.

28. The apparatus of claim 24, wherein heat left unused in said first analyzer column is supplied to said second analyzer column and said aldehyde column.

29. The apparatus of claim 24, wherein heat left unused in said dehydration unit is supplied to said first analyzer column.

30. The apparatus of claim 24, wherein said simmering column contains copper internals for effective removal of methanol and sulphurous impurities from said ethanol product streams.

31. The apparatus of claim 24, wherein not more than about 2 kilograms of steam is required to obtain 1 litre of ethanol product.

## Patentansprüche

1. Destillationsverfahren zur Abtrennung von Ethanol aus einem verdünntes Ethanol enthaltenden Ausgangsmaterial, umfassend:
(a) Teilen des Ausgangsmaterials in zwei Beschickungsströme ungleicher Größe;
(b) Vorwärmen sowohl des größeren als auch des kleineren der genannten Beschickungsströme;
(c) paralleles Einführen der genannten vorgewärmten Beschickungsströme in ein Paar Analysekolonnen;
(d) Aufrechterhalten eines höheren Drucks in der ersten Analysekolonne, die den genannten größeren Beschickungsstrom empfängt, als in der genannten zweiten Analysekolonne, die den genannten kleineren Beschickungsstrom empfängt;
(e) Entnehmen erster wässriger Ethanolströme aus dem oberen Teil der genannten Analysesäulen und ihr Einspeisen in eine Aldehydkolonne, um einen aldehydfreien Ethanolstrom an dem Bodenteil und einen Strom technischen Alkohols an dem oberen Teil der Säule abzutrennen;
(f) Beimischen von zwischen drei bis ungefähr zwölf Teilen Wasser oder verbrauchten Bodensatzes zu einem Teil Ethanol in den genannten aldehydfreien Ethanolstrom, sein Vorwärmen und sein Aussetzen einer Niedrigsiederkolonne, um einen zweiten wässrigen Ethanolstrom aus dem unteren Teil, und einen Strom technischen Alkohols aus dem oberen Teil der Kolonne abzutrennen;
(g) Vorwärmen des genannten zweiten wässrigen Ethanolstroms und sein Aussetzen einer Rektifikationskolonne, um einen rektifizierten Ethanolstrom zu erhalten, worin der genannte rektifizierte Ethanolstrom mit einem elementaren Hydroxid behandelt wird, um seinen pH auf zwischen pH 7 und pH 11 einzustellen;
(h) Dehydratisieren des genannten rektifizierten Ethanolstroms unter Verwendung einer Dehydratisierungseinheit, um einen wasserfreien Ethanolstrom zu erhalten, worin die genannte Dehydratisierung des rektifizierten Ethanolstroms mittels einer Membranseparationstechnik, Molekularsiebdehydratisierung oder Adsorptionsabtrennung von Wasser oder einer Kombination davon, erreicht wird;
(i) Einführen des genannten wasserfreien Ethanolstroms in eine Hochsiederkolonne, um einen Strom technischen Alkohols an dem Bodenteil und einen finalen wasserfreien Ethanolstrom an dem oberen Teil der Kolonne abzutrennen, worin der genannte wasserfreie Ethanolstrom mit einem anorganischen Oxidationsmittel bis zu einer Konzentration von 30 ppm behandelt wird, und
(j) Einführen des genannten finalen wasserfreien Ethanolstroms in eine Siedekolonne, um Ethanol-Produktströme durch optionales Hinzufügen von Wasser in verschiedenen Mengen zu dem genannten finalen wasserfreien Ethanolstrom zu erhalten.

2. Verfahren nach Anspruch 1, worin der genannte größere Beschickungsstrom zwischen 50 bis 70 Gewichts-% des gesamten Ausgangsmaterials umfasst.

3. Verfahren nach Anspruch 1, worin die genannte Aldehydkolonne wenigstens eine Aldehydkomponente abtrennt, die in den genannten ersten wasserfreien Ethanolströmen vorhanden ist, welche aus den genannten Analysekolonnen erhalten werden.

4. Verfahren nach Anspruch 1, worin die genannte Niedrigsiederkolonne wenigstens eine niedrig siedende Komponente abtrennt, die in dem genannten aldehydfreien Ethanolstrom enthalten ist, welcher aus der genannten Aldehydkolonne erhalten wurde.

5. Verfahren nach Anspruch 1, worin die genannte Rektifikationskolonne weiterhin den genannten zweiten wasserfreien Ethanolstrom, der aus der genannten Niedrigsiederkolonne erhalten wurde, von wenigstens einer hochsiedenden oder niedrigsiedenden Komponente oder beiden reinigt.

6. Verfahren nach Anspruch 1, worin die genannte Hochsiederkolonne wenigstens eine hochsiedende Komponente abtrennt, die in dem genannten wasserfreien Ethanolstrom vorliegt, welcher aus der genannten Dehydrationseinheit erhalten wurde.

7. Verfahren nach Anspruch 1, worin Wasser optional in dem letzten Schritt in verschiedenen Mengen zu erhaltenen Ethanolprodukten hinzugefügt wird, die ungefähr zwischen 0,1 Volumen-% bis 6 Volumen-% Wasser besitzen.

8. Verfahren nach Anspruch 1, worin nicht mehr als ungefähr 2 Kilogramm Dampf erforderlich sind, um ungefähr einen Liter Ethanolprodukt zu erhalten.

9. Verfahren nach Anspruch 1, worin in der genannten Rektifikationskolonne ungenutzt verbliebene Wärme in die genannte erste Analysenkolonne integriert wird.

10. Verfahren nach Anspruch 1, worin in der genannten Dehydrationseinheit ungenutzt verbliebene Wärme in die genannte erste Analysenkolonne integriert wird.

11. Verfahren nach Anspruch 1, worin in der genannten ersten Analysekolonne ungenutzt verbliebene Wärme in die genannte Aldehyd- oder die genannte zweite Analysekolonne integriert wird.

12. Verfahren nach Anspruch 1, worin in der genannten Niedrigsiederkolonne ungenutzt verbliebene Wärme in die genannte Hochsiederkolonne integriert wird.

13. Verfahren nach Anspruch 1, worin frische Energie in Form von Dampf der genannten Niedrigsiederkolonne, der genannten Rektifikationskolonne und der genannten Dehydrationseinheit bei Bedarf zugeführt wird.

14. Verfahren nach Anspruch 1, worin Vorwärmen bei verschiedenen Schritten durch Integrieren von Wärme erreicht wird, die bei den Schritten anderer Einheiten ungenutzt verblieben ist.

15. Verfahren nach Anspruch 1, worin das genannte, verdünntes Ethanol enthaltende Ausgangsmaterial umfasst: eine Gärflüssigkeit oder synthetisches Ethanol-Einsatzmaterial oder eine Kombination davon.

16. Verfahren nach Anspruch 1, worin die Menge an Ethanol in dem genannten, verdünntes Ethanol enthaltenden Ausgangsmaterial wenigstens 3 Volumenprozent ist.

17. Verfahren nach Anspruch 1, worin die genannten Ströme technischen Alkohols aus der genannten Aldehydkolonne, der genannten Niedrigsiederkolonne, der genannten Hochsiederkolonne und der genannten Siedekolonne erhalten werden.

18. Verfahren nach Anspruch 1, worin der genannte rektifizierte Ethanolstrom, der aus der genannten Rektifikationskolonne erhalten wird, wenigstens 93 Volumen-% Ethanol enthält.

19. Verfahren nach Anspruch 1, worin der genannte wasserfreie Ethanolstrom, der aus der genannten Dehydratationseinheit erhalten wird, wenigstens 99 Volumen-% Ethanol enthält.

20. Verfahren nach Anspruch 1, worin die genannten Ethanol-Produktströme, die aus der genannten Siedekolonne erhalten werden, nicht mehr als 1 ppm Methanol und nicht mehr als 1 ppm Aldehyd enthalten.

21. Verfahren nach Anspruch 1, worin die genannten Ethanol-Produktströme, die aus der genannten Siedekolonne erhalten werden, nicht mehr als 1 ppm Ethylacetat und nicht mehr als 2 ppm Fuselöle enthalten.

22. Verfahren nach Anspruch 1, worin Ethanol-Produktströme, die aus der genannten Siedekolonne erhalten werden, neutral in Geruch oder Geschmack sind.

23. Verfahren nach Anspruch 1, worin in den genannten ersten wässrigen Ethanolströmen, die aus den genannten Analysekolonnen erhalten werden, die Menge an Isopropylalkohol auf wenigstens 40 % der anfänglichen Menge, die in den genannten Einsatzströmen vorliegt, reduziert ist.

24. Destillationsvorrichtung zur Gewinnung von Ethanol aus einem verdünntes Ethanol enthaltenden Ausgangsmaterial, zur Verwendung in einem Verfahren nach einem der Ansprüche 1 bis 23, umfassend:
(a) eine Ausgangsmaterial-Aufteilungseinheit, um zwei Einsatzströme ungleicher Größe zu erhalten;
(b) eine Vorwärmeinheit, um sowohl den größeren als auch den kleineren der genannten Einsatzströme zu erwärmen;
(c) eine erste Analysekolonne und eine zweite Analysekolonne, eine Aldehydkolonne, eine Niedrigsiederkolonne, eine Rektifikationskolonne, eine Dehydrationseinheit, eine Hochsiederkolonne und eine Siedekolonne;
(d) eine Vorkehrung zum Einführen des größeren der genannten Einsatzströme in die genannte erste Analysekolonne, und eine Vorkehrung zum Einführen des kleineren der genannten Einsatzströme in die genannte zweite Analysekolonne;
(e) einen ersten Reboiler zum Kondensieren von Alkoholdämpfen aus der Dehydrationseinheit stromabwärts, um die Wärme bereitzustellen, die in der genannten ersten Analysekolonne benötigt wird;
(f) einen zweiten Reboiler, zum Kondensieren von Überkopfdämpfen aus einer Rektifikationskolonne stromabwärts der genannten Analysekolonnen, um die Wärme bereitzustellen, die in der genannten ersten Analysekolonne benötigt wird;
(g) einen dritten Reboiler, zum Kondensieren von Überkopfdämpfen aus der genannten ersten Analysekolonne, um die Wärme bereitzustellen, die in der genannten zweiten Analysekolonne benötigt wird;
(h) eine Vorkehrung zum Entnehmen erster wässriger Ethanolströme aus einem oberen Teil der genannten Analysekolonnen;
(i) eine Vorkehrung zum Vorwärmen und Einführen der genannten ersten wässrigen Ethanolströme in eine genannte Aldehydkolonne;
(j) Eine Vorkehrung zum Entnehmen eines aldehydfreien Ethanolstroms aus einem Bodenteil der genannten Aldehydkolonne;
(k) ein Verdünnungstank zum Verdünnen des genannten aldehydfreien Ethanolstroms mit Wasser oder verbrauchtem Bodensatz;
(l) eine Vorkehrung zum Vorwärmen und Einführen des genannten verdünnten, aldehydfreien Ethanolstroms in die genannte Rektifikationskolonne;
(m) eine Vorkehrung zum Entnehmen eines zweiten wässrigen Ethanolstroms aus dem unteren Teil, und zum Entnehmen eines Stroms technischen Alkoholstroms aus dem oberen Teil der genannten Niedrigsiederkolonne;
(n) eine Vorkehrung zum Vorwärmen und Einführen des genannten zweiten wässrigen Ethanolstroms in die genannte Rektifikationskolonne;
(o) eine Vorkehrung zum Entnehmen eines rektifizierten Ethanolstroms aus einem oberen Teil der genannten Rektifikationskolonne;
(p) eine Vorkehrung zum Einführen eines elementaren Hydroxids in den genannten rektifizierten Ethanolstrom, um den pH auf zwischen 7 und 11 einzustellen;
(q) eine Vorkehrung zum Einführen des genannten rektifizierten Ethanolstroms in die genannte Dehydrationseinheit, worin die genannte Dehydrationseinheit eine oder mehrere ist aus einer Membranseparationseinheit, Molekularsiebeinheit oder einer Adsorptionseinheit;
(r) eine Vorkehrung zum Entnehmen eines wasserfreien Ethanolstroms aus der genannten Dehydrationseinheit;
(s) eine Vorkehrung zum Einführen des genannten wasserfreien Ethanolstroms in die genannte Hochsiederkolonne;
(t) eine Vorkehrung zum Einführen eines anorganischen Oxidationsmittels in den genannten wasserfreien Ethanolstrom;
(u) eine Vorkehrung zum Abtrennen eines Stroms technischen Alkohols am Bodenteil, und eines finalen wasserfreien Ethanolstroms an dem oberen Teil der genannten Hochsiederkolonne;
(v) eine Vorkehrung zum Einführen des genannten wasserfreien Ethanolstroms in eine Siedekolonne, welche Mittel besitzt zum optionalen Mischen mit Wasser; und
(w) eine Vorkehrung zum Entnehmen von Ethanol-Produktströmen aus der genannten Siedekolonne.

25. Vorrichtung nach Anspruch 24, worin in der genannten Niedrigsiederkolonne ungenutzt verbliebene Wärme der genannten Hochsiederkolonne zugeführt wird.

26. Vorrichtung nach Anspruch 24, worin in der genannten Hochsiederkolonne ungenutzt verbliebene Wärme der genannten Rektifikationskolonne zugeführt wird.

27. Vorrichtung nach Anspruch 24, worin in der genannten Rektifikationskolonne ungenutzt verbliebene Wärme der genannten ersten Analysekolonne zugeführt wird.

28. Vorrichtung nach Anspruch 24, worin in der genannten ersten Analysekolonne ungenutzt verbliebene Wärme der genannten zweiten Analysekolonne und der genannten Aldehydkolonne zugeführt wird.

29. Vorrichtung nach Anspruch 24, worin in der genannten Dehydrationseinheit Rektifizierkolonne Wärme der genannten ersten Analysekolonne zugeführt wird.

30. Vorrichtung nach Anspruch 24, worin die genannte Siedekolonne Kupfereinbauten enthält zum effektiven Entfernen von Methanol und Schwefelverunreinigungen aus den genannten Ethanol-Produktströmen.

31. Vorrichtung nach Anspruch 24, worin nicht mehr als ungefähr 2 Kilogramm Dampf erforderlich sind, um 1 Liter Ethanolprodukt zu erhalten.

## Revendications

1. Procédé de distillation pour séparer l'éthanol d'une charge d'alimentation contenant de l'éthanol dilué, comprenant les étapes consistant :
(a) à diviser la charge d'alimentation en deux courants d'alimentation de taille inégale ;
(b) à préchauffer à la fois le plus grand et le plus petit desdits courants d'alimentation ;
(c) à introduire lesdits courants d'alimentation préchauffés en parallèle dans une paire de colonnes d'analyse ;
(d) à maintenir une pression plus élevée dans une première colonne d'analyse recevant ledit plus grand courant d'alimentation que dans une deuxième colonne d'analyse recevant ledit plus petit courant d'alimentation ;
(e) à prélever des premiers courants d'éthanol hydraté de la partie supérieure desdites colonnes d'analyse et à les alimenter dans une colonne d'aldéhyde pour séparer un courant d'éthanol exempt d'aldéhyde au niveau de la partie inférieure et un courant d'alcool technique au niveau de la partie supérieure de la colonne ;
(f) à mélanger entre environ trois et environ douze parties d'eau ou de lies épuisées à une partie d'éthanol dans ledit courant d'éthanol exempt d'aldéhyde, à les préchauffer et à les soumettre à une colonne à haut point d'ébullition pour séparer un deuxième courant d'éthanol hydraté de la partie inférieure et un courant d'alcool technique de la partie supérieure de la colonne ;
(g) à préchauffer ledit deuxième courant d'éthanol hydraté et à le soumettre à une colonne de rectification pour obtenir un courant d'éthanol rectifié, dans lequel ledit courant d'éthanol rectifié est traité avec un hydroxyde élémentaire pour ajuster son pH entre pH 7 et pH 11 ;
(h) à déshydrater ledit courant d'éthanol rectifié en utilisant une unité de déshydratation pour obtenir un courant d'éthanol anhydre, dans lequel ladite déshydratation du courant d'éthanol rectifié est obtenue au moyen d'une technique de séparation par membrane, d'une déshydratation sur tamis moléculaire ou d'une séparation par adsorption d'eau ou d'une combinaison de celles-ci ;
(i) à introduire ledit courant d'éthanol anhydre dans une colonne à haut point d'ébullition pour séparer un courant d'alcool technique au niveau de la partie inférieure et un courant d'éthanol anhydre final au niveau de la partie supérieure de la colonne ; dans lequel ledit courant d'éthanol anhydre est traité avec un agent oxydant inorganique jusqu'à une concentration de 30 PPM, et
(j) à introduire ledit courant d'éthanol anhydre final dans une colonne de mijotage pour obtenir des courants de produit d'éthanol en ajoutant facultativement de l'eau audit courant d'éthanol anhydre final en différentes quantités.

2. Procédé de la revendication 1, dans lequel ledit plus grand courant d'alimentation comprend entre 50 et 70% en poids de la charge d'alimentation totale.

3. Procédé de la revendication 1, dans lequel ladite colonne d'aldéhyde sépare au moins un composant d'aldéhyde présent dans lesdits premiers courants d'éthanol hydraté obtenus à partir desdites colonnes d'analyse.

4. Procédé de la revendication 1, dans lequel ladite colonne à bas point d'ébullition sépare au moins un composant à bas point d'ébullition présent dans ledit courant d'éthanol exempt d'aldéhyde obtenu à partir de ladite colonne d'aldéhyde.

5. Procédé de la revendication 1, dans lequel ladite colonne de rectification purifie en outre ledit deuxième courant d'éthanol hydraté obtenu à partir de ladite colonne à bas point d'ébullition d'au moins un composant à haut point d'ébullition ou un composant à bas point d'ébullition ou des deux.

6. Procédé de la revendication 1, dans lequel ladite colonne à haut point d'ébullition sépare au moins un composant à haut point d'ébullition présent dans ledit courant d'éthanol anhydre obtenu à partir de ladite unité de déshydratation.

7. Procédé de la revendication 1, dans lequel de l'eau est facultativement ajoutée dans l'étape finale en différentes quantités à des produits d'éthanol obtenus ayant approximativement entre 0,1% et 6% d'eau en volume.

8. Procédé de la revendication 1, dans lequel pas plus d'environ 2 kilogrammes de vapeur sont nécessaires pour obtenir environ un litre d'un produit d'éthanol.

9. Procédé de la revendication 1, dans lequel la chaleur non utilisée dans ladite colonne de rectification est intégrée à ladite première colonne d'analyse.

10. Procédé de la revendication 1, dans lequel la chaleur non utilisée dans ladite unité de déshydratation est intégrée à ladite première colonne d'analyse.

11. Procédé de la revendication 1, dans lequel la chaleur non utilisée dans ladite première colonne d'analyse est intégrée à ladite colonne d'aldéhyde ou à ladite deuxième colonne d'analyse.

12. Procédé de la revendication 1, dans lequel la chaleur non utilisée dans ladite colonne à bas point d'ébullition est intégrée à ladite colonne à haut point d'ébullition.

13. Procédé de la revendication 1, dans lequel de l'énergie fraîche sous forme de vapeur est fournie à ladite colonne à bas point d'ébullition, à ladite colonne de rectification et à ladite unité de déshydratation au besoin.

14. Procédé de la revendication 1, dans lequel le préchauffage à différentes étapes est obtenu en intégrant la chaleur non utilisée dans d'autres étapes unitaires.

15. Procédé de la revendication 1, dans lequel ladite charge d'alimentation contenant de l'éthanol dilué comprend : un wash fermenté ou une charge d'éthanol synthétique ou une combinaison de ceux-ci.

16. Procédé de la revendication 1, dans lequel la quantité d'éthanol dans ladite charge d'alimentation contenant de l'éthanol dilué est d'au moins 3 pour cent en volume.

17. Procédé de la revendication 1, dans lequel lesdits courants d'alcool technique sont obtenus à partir de ladite colonne d'aldéhyde, de ladite colonne à bas point d'ébullition, de ladite colonne à haut point d'ébullition et de ladite colonne de mijotage.

18. Procédé de la revendication 1, dans lequel ledit courant d'éthanol rectifié obtenu à partir de ladite colonne de rectification contient au moins 93% d'éthanol en volume.

19. Procédé de la revendication 1, dans lequel ledit courant d'éthanol anhydre obtenu à partir de ladite unité de déshydratation contient au moins 99% d'éthanol en volume.

20. Procédé de la revendication 1, dans lequel lesdits courants de produit d'éthanol obtenus à partir de ladite colonne de mijotage comprennent du méthanol à une teneur ne dépassant pas 1 PPM et de l'aldéhyde à une teneur ne dépassant pas 1 PPM.

21. Procédé de la revendication 1, dans lequel lesdits courants de produit d'éthanol obtenus à partir de ladite colonne de mijotage comprennent de l'acétate d'éthyle à une teneur ne dépassant pas 1 PPM et des huiles de fusel à une teneur ne dépassant pas 2 PPM.

22. Procédé de la revendication 1, dans lequel les courants de produit d'éthanol obtenus à partir de ladite colonne de mijotage sont neutres en odeur ou en goût.

23. Procédé de la revendication 1, dans lequel dans lesdits premiers courants d'éthanol hydraté obtenus à partir desdites colonnes d'analyse, la quantité d'alcool isopropylique est réduite à au moins 40% de la quantité initiale présente dans lesdits courants d'alimentation.

24. Appareil de distillation permettant de récupérer de l'éthanol à partir d'une charge d'alimentation contenant de l'éthanol dilué, pour une utilisation dans un procédé de l'une des revendications 1 à 23, comprenant :
(a) une unité de division de charge d'alimentation pour obtenir deux courants d'alimentation de taille inégale ;
(b) une unité de préchauffage pour chauffer à la fois le plus grand et le plus petit desdits courants d'alimentation ;
(c) une première colonne d'analyse et une deuxième colonne d'analyse, une colonne d'aldéhyde, une colonne à bas point d'ébullition, une colonne de rectification, une unité de déshydratation, une colonne à haut point d'ébullition et une colonne de mijotage ;
(d) une disposition pour introduire le plus grand desdits courants d'alimentation dans ladite première colonne d'analyse et une disposition pour introduire le plus petit desdits courants d'alimentation dans ladite deuxième colonne d'analyse ;
(e) un premier rebouilleur pour condenser les vapeurs d'alcool provenant de l'unité de déshydratation en aval pour fournir la chaleur requise dans ladite première colonne d'analyse ;
(f) un deuxième rebouilleur pour condenser les vapeurs de tête provenant d'une colonne de rectification en aval desdites colonnes d'analyse pour fournir la chaleur requise dans ladite première colonne d'analyse ;
(g) un troisième rebouilleur pour condenser les vapeurs de tête provenant de ladite première colonne d'analyse pour fournir la chaleur requise dans ladite deuxième colonne d'analyse ;
(h) une disposition pour prélever des premiers courants d'éthanol hydraté d'une partie supérieure desdites colonnes d'analyse ;
(i) une disposition pour préchauffer et introduire lesdits premiers courants d'éthanol hydraté dans ladite colonne d'aldéhyde ;
(j) une disposition pour prélever un courant d'éthanol exempt d'aldéhyde d'une partie inférieure de ladite colonne d'aldéhyde ;
(k) un réservoir de dilution pour la dilution dudit courant d'éthanol exempt d'aldéhyde avec de l'eau ou des lies épuisées ;
(l) une disposition pour préchauffer et introduire ledit courant d'éthanol exempt d'aldéhyde dilué dans ladite colonne de rectification ;
(m) une disposition pour prélever un deuxième courant d'éthanol hydraté de la partie inférieure et pour prélever un courant d'alcool technique de la partie supérieure de ladite colonne à bas point d'ébullition ;
(n) une disposition pour préchauffer et introduire ledit deuxième courant d'éthanol hydraté dans ladite colonne de rectification ;
(o) une disposition pour prélever un courant d'éthanol rectifié d'une partie supérieure de ladite colonne de rectification ;
(p) une disposition pour introduire un hydroxyde élémentaire dans ledit courant d'éthanol rectifié pour ajuster son pH entre 7 et 11 ;
(q) une disposition pour introduire ledit courant d'éthanol rectifié dans ladite unité de déshydratation, dans lequel ladite unité de déshydratation est une ou plusieurs parmi une unité de séparation par membrane, une unité de tamis moléculaire et une unité d'adsorption ;
(r) une disposition pour prélever un courant d'éthanol anhydre de ladite unité de déshydratation ;
(s) une disposition pour introduire ledit courant d'éthanol anhydre dans ladite colonne à haut point d'ébullition ;
(t) une disposition pour introduire un agent oxydant inorganique dans ledit courant d'éthanol anhydre ;
(u) une disposition pour séparer un courant d'alcool technique au niveau de la partie inférieure et un courant d'éthanol anhydre final au niveau de la partie supérieure de ladite colonne à haut point d'ébullition ;
(v) une disposition pour introduire ledit courant d'éthanol anhydre dans une colonne de mijotage ayant des moyens pour mélanger facultativement de l'eau ; et
(w) une disposition pour prélever les courants de produit d'éthanol de ladite colonne de mijotage.

25. Appareil de la revendication 24, dans lequel la chaleur non utilisée dans ladite colonne à bas point d'ébullition est fournie à ladite colonne à haut point d'ébullition.

26. Appareil de la revendication 24, dans lequel la chaleur non utilisée dans ladite colonne à haut point d'ébullition est fournie à ladite colonne de rectification.

27. Appareil de la revendication 24, dans lequel la chaleur non utilisée dans ladite colonne de rectification est fournie à ladite première colonne d'analyse.

28. Appareil de la revendication 24, dans lequel la chaleur non utilisée dans ladite première colonne d'analyse est fournie à ladite deuxième colonne d'analyse et à ladite colonne d'aldéhyde.

29. Appareil de la revendication 24, dans lequel la chaleur non utilisée dans ladite unité de déshydratation est fournie à ladite première colonne d'analyse.

30. Appareil de la revendication 24, dans lequel ladite colonne de mijotage contient des éléments internes en cuivre pour l'élimination efficace du méthanol et des impuretés sulfureuses desdits courants de produit d'éthanol.

31. Appareil de la revendication 24, dans lequel pas plus d'environ 2 kilogrammes de vapeur sont nécessaires pour obtenir 1 litre de produit d'éthanol.
